Europäisches Patentamt

European Patent Office

Office européen des brevets

(19)

(11) Publication number: **0 247 258 B1**

# EUROPEAN PATENT SPECIFICATION

(12)

(45) Date of publication of patent specification: 06.03.91

(51) Int. Cl.5: **C12N 9/64**, C07K 3/02, C07K 3/18, C07K 15/06, //A61K37/553,A61K37/64

(21) Application number: 86304066.3

(22) Date of filing: 28.05.86

(54) Process for concentrating and separating trypsin inhibitor and kallidinogenase in human urine.

(43) Date of publication of application:
02.12.87 Bulletin 87/49

(45) Publication of the grant of the patent:
06.03.91 Bulletin 91/10

(84) Designated Contracting States:
AT BE CH DE FR GB IT LI LU NL SE

(56) References cited:

CHEMICAL ABSTRACTS, vol. 104, no. 18, 5th May 1986, page 450, abstract no. 155950z, Columbus, Ohio, US & JP-A-60 260 518 (Nippon Chemical Research K.K.), 23.12.85

ARCH. BIOCHEM. BIOPHYS., vol. 55, 1955, pages 286,287; D.R. CELANDER et al.: "The application of foam technique to the partial purification of a urine activator of plasma profibrinolysin"

(73) Proprietor: NIHON CHEMICAL RESEARCH KABUSHIKI KAISHA also known as JAPAN CHEMICAL RESEARCH CO., LTD
4-20, Mikagehommachi 3-chome
Higashinada-ku
Kobe Hyogo 658(JP)

(72) Inventor: Yuki, Yoshikazu
1-2, Mikatadai 4-chome Nishi-ku
Kobe Hyogo 673(JP)
Inventor: Nakanishi, Koichiro
2-3-324, Takahama-cho
Ashiya Hyogo 659(JP)
Inventor: Hiratani, Hajime
705-3, Tottori Hannan-cho
Sennan-gun Osaka 599-02(JP)
Inventor: Sawa, Kayoko
9-7, Iazono-cho
Ashiya Hyogo 659(JP)

(74) Representative: Smart, Peter John et al
W.H. BECK, GREENER & CO 7 Stone Buildings Lincoln's Inn
London WC2A 3SZ(GB)

Rank Xerox (UK) Business Services

## Description

Process for concentrating and separating trypsin inhibitor and kallidinogenase in human urine

This invention relates to a process of producing human urine trypsin inhibitor (hereinafter referred to briefly as "HUTI") and human urine kallidinogenase (hereinafter referred to briefly as "HUKN") on an industrial scale and in improved yields by concentrating simultaneously HUTI and HUKN contained in urine.

(Prior Art)

Urine trypsin inhibitors which are a kind of enzymatic activity inhibitor contained in urines of mammarians are known to vary in characteristic properties depending upon the species of animals from which they are derived [Carlson, Enzyme, 18 , 176 (1974)]. HUTI, a glucoprotein having a molecular weight of 68,000 and an isoelectric point of pH 2 to 3, inhibits particularly trypsin, chymotrypsin, plasmin [Sumi, et al., Journal of Physiol. of Japan, 39 , 53 (1977)], human erastase [Johnson et al., Hoppe Seyler's Z. Physiol. Chem., 363 , 1167 (1982)], etc., and is possibly considered to find application as an anti-inflammatory agent. On the other hand, kallidinogenase is a proteolytic enzyme produced in every part of living bodies of animals, and, through its decomposition of kininogen in serum to produce kinin, demonstrates diverse activites, such as blood pressure decreasing and blood flow increasing activities. Both of these are hithertofore separated from different species of animals from man, and an attempt has been made to utilize them as a drug. Especially, kallidinogenase has been produced so far using porcine pancreas and urine as a raw material. However, these are a protein foreign to man and exhibit antigenicity; when they are used clinically, their frequent administration often produces anaphylaxis, and the problem has been encountered from the standpoint of safety. In contrast to this, HUTI and HUKN are proteins of human origin, and consequently, are entirely free from the above side effect to human body.

As the conventional concentration purification method for HUTI, there may be mentioned, for example, the precipitation method involving the combined use of trichloroacetic acid, ammonium sulfate and methanol [N. R. Shulman: J.B.C., 213 , 655 (1955)], the method utilizing bentonite[T. A. Strup: Scan. J. Clin. Lab. Invest., 11 , 181 (1959)] and the method using DEAE-cellulose [G. J. Proksch: J. Lab & Clin. Med., 79 , 491 (1972)]. With reference to the conventional concentration purification method for HUKN, there are known, for example, the method using silica gel (Japanese Patent Application laid-open No. 99151/1980) and the method utilizing a microporous type of ion exchange resin, such as WA-30 (Japanese Patent Application laid-open No. 5515/1975) as well as the concentration purification method for HUKN developed by the present inventors (Japanese Patent Application laid-open No. 6883/1984) which consists of using chitosan as an adsorbent. As the method of concentrating simultaneously both of HUTI and HUKN, furthermore, there is known the method by Sumi et al. [Sumi, et al.: Medicine and Biology ("Igaku to Seibutsugaku", vol. 100 (1) (1980)] which involves the use of Arginine-Sepharose. Referring to the above-mentioned methods, those intended for use in the purification and processing into preparations of either HUTI or HUKN alone are not always satisfactory in terms of cost incurred and operation involved, while the method of concentrating simultaneously both of these requires too much expensive adsorbent and too complex operational procedure to be conducted into practice on an industrial scale.

The present inventors hit upon an idea to performing bubbling by taking advantage of the fact that HUTI and HUKN contained as an active ingredient in urine are amphoteric proteins, followed by allowing both to rest on bubbles to thereby carry out concentration. The method has been utilized for concentrating urokinase, a urine enzyme (Celander, DR, Langlinais,R. P. and Guest, M. M.; Arch. Biochem. Biophys., 55 , 286 (1955)), but has never been known as a method of concentrating urine kallikrein and urine trypsin inhibitor.

Such being the case, bubbling was carried out at different pH values to concentrate urine, and the concentrated urines were comparatively investigated for the recovery rates of HUKN and HUTI.

In determining the pH of urine to be tested, the following two factors were taken into consideration:
(1) HUTI undergoes degradation into fractions with smaller molecular weights, when maintained at a pH of below 5.0.
(2) HUKN is unstable at a pH of below 4.0.

Taking the above into account, 60 ℓ of the urine of a lot was divided into 10-ℓ portions, which were adjusted with 6N-HC1 or 6N-NaOH to a pH 5.0 to 10.0 (precipitates, whenever they separated out, were removed) and then filled into respective colums of l4cm in diameter x 100 cm in height (which each had a porous plate of 5 mm in diameter provided at the lower flange); air was introduced into the columns by means of a compressor-pump to generate fine bubbles, and the bubbles overflown out of the columns were collected, followed by recovery of about 2 ℓ of the concentrated urine; and, the recovery ratios for HUTI and HUKN were calculated, with the results being obtained as shown in Table 1.

### Table 1: Yields in relation to pH value

| pH | Yield | |
|---|---|---|
| | HUTI | HUKN |
| 5 | 62 % | 58 % |
| 6 | 74 % | 78 % |
| 7 | 85 % | 88 % |
| 8 | 69 % | 71 % |
| 9 | 58 % | 53 % |
| 10 | 40 % | 38 % |

Note: The urines, at a pH of above 8.0, produced precipitates of mucopolysaccharides, and after the precipitates were removed, bubbling was effected.

The recovery ratios were calculated on the basis of the HUTI and HUKN activities in the original urine without pH adjustment being taken as 100 %.

The above results indicate that when bubbling is effected at a pH value of 6.0 to 8.0, preferably in the neighborhood of 7.0 and concentration is completed at a point of time where the concentrate corresponds to one-fifth the volume of the raw original urine, there can be recovered HUTI and HUKN in a efficient way (under these conditions, uropepsin was not concentrated, but was found to be present at the same level of concentration as in the raw urine).

The present inventors conducted further research, whereupon comprative investigation was performed, by the following procedure and with use of such concentrated urines, on a variety of inorganic adsorbents, ion exchange materials and protein coagulants for the purification degree through adsorption for HUTI and HUKN.

In treating the concentrated urine with each of the above materials,
(1) 2 % of the concentrated urine was added to each of the materials, and
(2) The pH was maintained at a pH value of above 5.0 in order to prevent HUTI from degradation into lower-molecular-weight fractions by uropepsin being present as well in the concentrated urine.

By the procedure similar to that as described above, 75 ℓ of urine was concentrated to give 15 ℓ of the concentrated urine (whereby the recovery ratios for HUTI and HUKN were found to be 84 % and 89 %, respectively). The concentrated urine was divided into 1-ℓ portions, which were subjected to adsorption with 20 g each of the materials, followed by elution. The eluates were dialyzed adequately with water, and the amounts of HUTI, HUKN and protein in the dialyzed eluates were determined. The recovery ratio based on the concentrated urine, along with specific activity, was calculated, with the results being shown in Table 2.

Table 2: Screening of adsorption materials for HUTI and HUKN

| No. | Adsorbent | Tested pH | Eluting liquid | Yield, % | | Spec.activity* | | Supplier |
|-----|-----------|-----------|----------------|----------|----------|----------------|----------|----------|
| | | | | HUTI | HUKN | HUTI | HUKN | |
| 1 | Kaolin | 5.0 | 2% aq. ammonia | 4 | 32 | 128 | 6.5 | Wako[1] |
| 2 | Bentonite | 5.0 | " | 63 | 3 | 150 | 2.3 | " |
| 3 | Acid clay | 5.0 | " | 34 | 28 | 121 | 3.1 | " |
| 4 | Silica gel | 5.0 | " | 0 | 51 | - | 9.0 | " |
| 5 | Activated alumina | 5.0 | " | 5 | 10 | 102 | 3.2 | " |
| 6 | Chitosan | 5.0 | " | 71 | 83 | 311 | 8.7 | Kyowa[2] |
| 7 | Amberlite XAD-7 | 7.0 | " | 30 | 21 | 77 | 3.4 | Rohm & Haas |
| 8 | DEAE-cellulose | " | $0.1M H_3PO_4$ -0.5M NaCl pH 7.5 | 51 | 55 | 146 | 4.8 | Braun & Co. (W.G.) |
| 9 | DEAE-Sephadex | " | " | 42 | 60 | 121 | 4.3 | Pharmacia Japan |
| 10 | QAE-Sephadex | " | " | 62 | 41 | 159 | 3.3 | " |
| 11 | Arginine-Sepharose | " | 2 % aq. ammonia | 62 | 60 | 205 | 6.6 | " |

Note: *), in unit/mg (protein)
    1), Wako Pure Chemical Industries, Ltd. of Japan
    2), Kyowa Oils & Fats Ind., Ltd. of Japan

On the basis of the above-described results, it was found that the adsorbent capable of recovering HUTI and HUKN in yields of 60 % simultaneously includes chitosan under No. 8 and arginin-Sepharose® under No. 15. With regard to the adsorbent of chitosan, the present inventors have filed an application for patent (Japanese Patent Application laid-open No. 6883/1984) covering its use for adsorption of HUKN, and it was furthermore found on the basis of the above results that adjustment at pH 5.0 of the concentrate through bubbling and addition of chitosan in amounts of not less than 2 % (W/V) can permit HUTI as well as HUKN to be extracted in improved yields. In return for this, nevertheless, the specific activity of HUKN is decreased down to about one-fourth, which means that conversely, HUTI contained in large amounts in urine was simultaneously adsorbed onto chitosan, followed by elution.

The eluate obtained after treatment with chitosan as described above was examined for the heat-stability under conditions of 60°C for 10 hours.

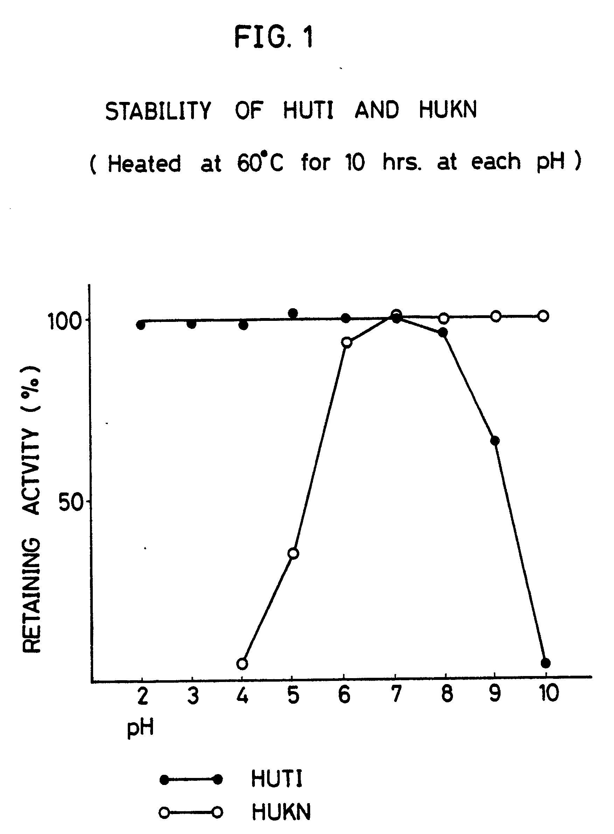

Fig. 1 is graphs representing respectively the HUTI and HUKN activities retained for the eluate when adjusted at a pH of 2 to 10 and heated at such adjusted pH. at 60°C for 10 hours.

As is evident from Fig. 1, HUTI and HUKN in the extract obtained by treatment with chitosan of the concentrated urine through bubbling both exhibit the maximal heat stability under the conditions of 60°C and 10 hours only at a pH value of 6 to 8, preferably in the neighborhood of 7.0.

The heat treatment under the conditions of 60°C and 10 hours has been conventionally adopted for virus sterilization of human urine urokinase preparations.

To subject the extract of this invention to such a heat treatment means to perform simultaneous virus sterilization of HUTI and HUKN, and this provides great advantages in processing both of them into preparations.

This invention is based on the above findings, and is directed to a process which comprises allowing human urine to bubble at a pH in the neighborhood of neutrality, collecting the resulting bubbles to concentrate simultaneously trypsin inhibitor and kallidinogenase in human urine, adjusting the concentrate to weak acidity, contacting the acidified concentrate with chitosan to allow the both components to be adsorbed simultaneously onto chitosan, subsequently eluting the both components simultaneously from the adsorbent with aqueous ammonia solution, neutralizing the eluate, and heat-treating the netralized eluate at about 60°C for about 10 hours, followed by separating the both components from the heat-treated eluate, if desired.

In performing concentration by means of the bubbling method, it is desirable to adjust human urine at a pH value of 6.0 to 8.0, preferably at pH 7.0, and it is advisable to concentrate human urine to a concentrated volume of about one-fifth that of the raw, original urine. The concentrate, which is to be contacted with chitosan, is desirably adjusted at a pH value of 4.5 to 6.5, preferably at pH 5.5. It is preferred to use chitosan in the amount of 20 to 30 g per liter of the concentrate, namely 2 to 3 % (w/v), preferably about 25 g, namely about 2.5 % (w/v).

HUTI and HUKN in the concentrate are selectively adsrobed onto chitosan through contacting with chitosan. The adsorbent material is collected by filtration and washed with water, if necessary, and HUTI and HUKN are desirably eluted in the pH range of 10.5 to 12.0 with use of 2 to 3N aqueous ammonia.

If desired, the resulting eluate is adjusted to a pH value of 6 to 8, preferably at pH 7.0, with 6N HCl and heat-treated at 60°C for 10 hours, and both components can be separated from each other and collected by means of such affinity chromatography as aprotinin-affinity chromatography and trypsin-affinity chromatography.

According to this invention, HUTI and HUKN can be concentrated efficiently by the bubbling method, whereupon chitosan is an adsorbent capable of selectively adsorbing both of the components in the resulting concentrated urine at a specifically stated pH value, and 2N to 3N aqueous ammonia acts as an eluting liquid for simultaneously desorbing both components. Heat treatment of the resulting eluate at a pH value in the neighborhood of neutrality at 60°C for 10 hours serves an effective purpose of producing the virus sterilization effect for processing into preparations. These actions cooperate with each other to permit both components in human urine to be concentrated and separated in a simplified manner.

According to this invention, there is provided a process wherein HUTI and HUKN, physiologically active substances in human urine, are concentrated and purified simultaneously and efficiently on an industrial scale. The heat-treatment at about 60°C for about 10 hours performed in the process to make the concentrated or purified material virus-free facilitates the subsequent processing into preparations. Therefore, it is an outstanding economiclal process for handling directly a large amount of urine.

Below described are the examples to illustrate this invention in more detail.

Example 1

A 10-liter portion of urine from a healthy male adult is adjusted to pH 7.0 and added into a column (which has a porous plate of 5 mm in diameter provided at the lower flange of the column) of 14 cm in diamter and 100 cm in height, and air is introduced by a compressor-pump into the column from the bottom end to perform bubbling. The resulting bubbles are received in another vessel, and at the time when the volume of the liquid produced after the bubbles have crushed reaches about 2 ℓ, the bubbling is completed to make the urine concentrate. 2N-HCl is added to the concentrate to adjust the pH to 5.0, and 50 g of chitosan is added to it, followed by stirring for 1 hour to adsorb HUTI and HUKN. The chitosan is collected by filtration and washed with water, and both components are eluted with 2N aqueous ammonia to give 150 ml of the eluate. The resulting eluate is adjusted to pH 6.5 with 6N-HCl and heat-treated at 60°C for 10 hours to give 148 ml of the treated liquid. The liquid is found to exhibit total HUTI activity of 47,200 inhibitory units (recovery ratio of 61 %) and purity of 310 inhibitory units/mg (protein), along with total HUKN activity of 1,245 units (recovery ratio of 70 %) and purity of 8.2. units/mg (protein).

Example 2

A 20-liter portion of urine from a healthy male adult is adjusted to pH 6.5, and filled into a column of 20 cm in diameter and 100 cm in height having a porous plate similar to that of Example 1, followed by bubbling. The resulting bubbles are received in another vessel, and at a time when about 4 ℓ of the liquid is collected, the bubbling is completed to produce the urine concentrate. 2N-HCl is added to the concentrated urine to adjust to pH 5.5, and 100 g of chitosan is added to it, followed by stirring for 1 hour to adsorb HUTI and HUKN. The chitosan is collected by filtration and washed with water, and the adsorbed substances are eluted with 3N aqueous ammonia. The resulting eluate of 300-ml volume is adjusted to pH 7.0 with 6N-HCl and heat-treated at 60°C for 10 hours to give 297 ml of the treated liquid. The liquid, when determined for the HUTI activity, is found exhibit total HUTI activity of 95,200 inhibitory units (recovery ratio of 60 %) and

5

purity of 302 inhibitor units/mg (protein), while determination of the HUKN activity shows that it possesses total HUKN activity of 2,789 units (recovery ratio of 72 %) and purity of 8.9 units. The liquid is not observed to show any decrease in activities of both active substances, even after stored at 5° C (in a refrigerator) for 1 week.

Example 3

The heat-treated extract liquid as obtained in Example 2 is dialyzed against 0.5M sodium chloride-0.1M sodium hydrogencarbonate buffer pH 8.0, and passed through a column consisting of an aprotinin-Sepharose ® (refer to the note 1) column (with a diameter of 3.0 cm and a height of 15 cm) equilibrated with the same buffer being connected with a trypsin-Sepharose (refer to the note 1) column (with a diameter of 3.0 cm and a height of 15 cm). The aprotinin-Sepharose column is subjected to elution with 0.1M acetic acid buffer (pH 3.5) containing 0.5M sodium chloride to give 41 ml of the HUKN eluate. The trypsin-Sepharose® column is subjected to elution with glycine hydrochloric acid buffer pH 2.6 containing 0.5M sodium chloride to give 48 ml of the HUTI eluate. The eluate exhibits total HUTI activity of 74,580 inhibitory units (recovery ratio of 47 %) and purity of 1,1320 inhibitory units/mg (protein), being entirely free from HUKN. The HUKN eluate shows total HUKN activity of 2,130 units (recovery ratio of 55 %) and purity of 825 units/mg (protein), being entirely free from HUTI.

Example 4

By carrying out the treatment similar to that as described in Example 2, there was obtained 309 ml of heat-treated extract, which exhibited total HUTI activity of 93,100 inhibitory units (recovery ratio of 63 %) and purity of 311 inhibitory units/mg (protein), along with total HUKN activity of 2,698 units (recovery ratio of 73 %) and purity of 9.0 units/mg (protein). The extract is dialyzed against 0.5M sodium chloride-0.05M phosphate buffer pH 8.5, and the dialyzate is passed through an aprotinin-Sepharose® column and trypsin-Sepharose column,as described in Example 3, in the mentioned order but after being equilibrated adequately with the above-described buffer. The aprotinin-Sepharose column is subjected to elution with 0.1M acetic acid buffer (pH 3.5) containing 0.5M sodium chloride to give 46 ml of the HUKN eluate. The trypsin-Sepharose column is subjected to elution with glycine hydrochloric acid buffer (pH 2.3) containing 0.5M sodium chloride to give 52 ml of the HUTI eluate. The HUKN eluate exhibits total HUKN activity of 2,053 units (recovery ratio of 53 %) and purity of 831 units/unit (protein, being entirely free from HUTI. The HUTI eluate shows total HUTI activity of 66,500 inhibitory units (recovery ratio of 45 %) and purity of 1,290 inhibitory units/mg (protein), being totally free from HUKN. The HUTI and HUKN eluates thus obtained are both found to show no decrease in titer, even after being stored at 4° C for 2 weeks subsequent to neutralization and sterile filtration.

In Examples 1 to 4, the HUTI inhibitory unit is determined by means of the method of Tanaka et al. [Biochimica et Biophysica Acta, 705 , 192 (1982)]; the employed trypsin is bovine trypsin type I manufactured by Sigma Co.

As to the HUKN inhibitory unit, the HUKN extracted for purification from human urine by the present inventors is subjected to dtermination of the inhibitory units by means of the vasodilator assay (J. Biochemistry, 58 , 201 (1965)], and using the HUKN as a standard, HUKN inhibitory units are determined by the fluorescent synthetic substrate method [J. Biochemistry, 82 , 1495 (1977)].

The protein amount is determined by use of the Lowry-Folin method using bovine serum albumin as a standard. [Note 1]:
Preparation of trypsin-Sepharose and aprotinin-Sepharose®:

Trypsin-Sepharose and aprotinin-Sepharose are prepared from trypsin type I [Sigma Co. of U.S.A.], aprotinin [Choay Co. of France] and CNBR activated Sepharose 4B [Pharmacia Japan] by means of P. Cuatrecasas' method [J. Biol. Chem., 245 , 3095 (1970)].

**Claims**

1. A method of concentrating and optionally separating human urine trypsin inhibitor and human urine kallidinogenase, characterised in that the method comprises forming bubbles in human urine at a pH in the neighbourhood of neutrality, collecting the resulting bubbles to concentrate simultaneously trypsin inhibitor and kallidinogenase in human urine, adjusting the concentrate to weak acidity, contacting the acidified concentrate with chitosan to allow the both components to be absorbed simultaneously onto chitosan, subsequently eluting both components simultaneously from the absorbent with aqueous ammonia solution, neutralizing the eluate, and heat-treating the neutralized eluate at about 60°C for about 10 hours, optionally followed by separating one or both said components from the heat-treated eluate.

2. A process as claimed in Claim 1, wherein human urine is bubbled at a pH of 6.0 to 8.0, preferably at pH 7.

3. A process as claimed in Claim 1 or Claim 2 wherein the concentrate is adjusted to a pH of 4.5 to 6.5, preferably to pH 5.5, for contacting with chitosan.

4. A process as claimed in any preceding claim, wherein the aqueous ammonia solution has a pH of 10.5 to 12.0.

5. A process as claimed in any preceding claim, wherein the concentrate is contacted with not less than 2% (w/v), preferably 2.5% (w/v), of chitosan.

6. A process as claimed in any preceding claim, wherein the eluate is neutralized to a pH of 6.0 to 7.5, preferably to pH 7, and then subjected to said heat treatment.

7. A process as claimed in any preceding claim, wherein the separation of both said components is performed by means of affinity chromatography.


**Revendications**

1. Procédé de concentration et éventuellement de séparation de l'inhibiteur de trypsine de l'urine humaine et de la Kallidinogénase de l'urine humaine, caractérisé en ce qu'il comprend les opérations consistant à former des bulles dans de l'urine humaine à un pH au voisinage de la neutralité à recueillir les bulles résultantes pour concentrer simultanément l'inhibiteur de trypsine et la kallidinogénase dans l'urine humaine, à régler une faible acidité du concentré, à mettre le concentré acidifié en contact avec du chitosane pour permettre que les deux composants soient adsorbés simultanément sur le chitosane, puis à séparer simultanément les deux compasants de l'adsorbant par élution avec une solution aqueuse d'ammoniac, à neutraliser l'éluat et à traiter par la chaleur l'éluat neutralisé à 60°C environ pendant 10 h environ, ce traitement étant éventuellement suivi de la séparation de l'un ou de l'un et l'autre desdits composants d'avec l'éluat traité par la chaleur.

2. Procédé selon la revendication 1, dans lequel les bulles sont formées dans l'urine humaine à un pH de 6,0 à 8,0, de préférence à un pH de 7.

3. Procédé selon la revendication 1 ou 2, dans lequel le concentré est réglé à un pH de 4,5 à 6,5, de préférence à un pH de 5,5, en vue de sa mise en contact avec le chitosane.

4. Procédé selon l'une quelconque des revendications 1 à 3, dans lequel la solution aqueuse d'ammoniac a un pH de 10,5 à 12,0.

5. Procédé selon l'une quelconque des revendications 1 à 4, dans lequel le concentré est mis en contact avec une quantité de chitosane non inférieure à 2% (p/v), de préférence non inférieure à 2,5% (p/v).

6. Procédé selon l'une quelconque des revendications 1 à 5, dans lequel l'éluat est neutralisé à un pH de 6,0 à 7,5, de préférence à un pH de 7, puis est soumis audit traitement thermique.

7. Procédé selon l'une quelconque des revendications 1 à 6, dans lequel la séparation desdits deux composants est effectuée par chromatographie d'affinité.

**Ansprüche**

1. Verfahren zum Konzentrieren und gegebenenfalls zum Separieren eines Humanurin-Trypsininhibitors und von Humanurin-Kallidinogenase, dadurch gekennzeichnet, daß das Verfahren die Bildung von Blasen im Humanurin bei einem pH in der Nähe der Neutralität, Sammeln der sich ergebenden Blasen zum gleichzeitigen Konzentrieren des Trypsininhibitors und der Kallidinogenase in Humanurin, Einstellen des Konzentrats auf schwache Acidität, Kontaktieren des angesäuerten Konzentrats mit Chitosan, damit die beiden Komponenten gleichzeitig auf dem Chitosan adsorbiert werden können, nachfolgendes gleichzeitiges Eluieren beider Komponenten von dem Adsorptionsmittel mit wäßriger Ammoniaklösung, Neutralisieren des Eluats und Hitzebehandeln des neutralisierten Eluats über etwa 10 Stunden bei etwa 60°C, gegebenenfalls gefolgt vom Separieren einer oder beider dieser Komponenten von dem hitzebehandelten Eluat, umfaßt.

2. Verfahren nach Anspruch 1, bei dem Humanurin bei einem pH von 6,0 bis 8,0, vorzugweise bei pH 7, durchsprudelt wird.

3. Verfahren nach Anspruch 1 oder 2, bei dem das Konzentrat zum Kontaktieren mit Chitosan auf einen pH von 4,5 bis 6,5, vorzugsweise auf pH 5,5 zum Kontaktieren mit dem Chitosan eingestellt wird.

4. Verfahren nach einem der vorhergehenden Ansprüche, bei dem die wäßrige Ammoniaklösung einen pH von 10,5 bis 12,0 hat.

5. Verfahren nach einem der vorhergehenden Ansprüche, bei dem das Konzentrat mit nicht weniger als 2 % (Gew./Vol.), vorzugsweise 2,5 % (Gew./Vol.), Chitosan kontaktiert wird.

6. Verfahren nach einem der vorhergehenden Ansprüche, bei dem das Eluat auf einen pH von 6,0 bis 7,5, vorzugsweise auf pH 7. neutralisiert und danach der Hitzebehandlung unterzogen wird.

7. Verfahren nach einem der vorhergehenden Ansprüche, bei dem die Separation der beiden Komponenten mit Hilfe der Affinitätschromatographie durchgeführt wird.

# FIG. 1

## STABILITY OF HUTI AND HUKN

### ( Heated at 60°C for 10 hrs. at each pH )